# EUROPÄISCHE PATENTSCHRIFT

(11) **EP 2 119 462 B1**
(45) Veröffentlichungstag und Bekanntmachung des Hinweises auf die Patenterteilung: **04.07.2012**
(21) Anmeldenummer: 09160356.3
(22) Anmeldetag: 15.05.2009
(51) Int. Cl.: A61M 5/158, A61M 5/32, A61M 25/06

(54) **Kanülenanordnung**
Cannula assembly
Agencement de canule

(30) Priorität: 15.05.2008 DE 102008002854
(43) Veröffentlichungstag der Anmeldung: 18.11.2009
(73) Patentinhaber: Süddeutsche Feinmechanik GmbH, 63607 Wächtersbach (DE)
(72) Erfinder: Kruse, Reinhard, 63571, Gelnhausen (DE); Kehr, Markus, 63599, Biebergemünd (DE)
(74) Vertreter: Stoffregen, Hans-Herbert

(56) Entgegenhaltungen:
- EP-A- 1 256 355
- WO-A-2005/000377
- WO-A-2005/120624
- FR-A- 2 847 478
- US-A- 5 405 332

## Beschreibung

Die Erfindung bezieht sich auf eine Kanülenanordnung umfassend eine Kanüle mit einem proximalen Abschnitt und einem distalen Abschnitt, die zueinander einen rechten oder einen im Wesentlichen rechten Winkel einschließen, eine von dem proximalen Abschnitt ausgehende Handhabe umfassend einen den proximalen Abschnitt aufnehmenden Mittelabschnitt mit von diesem ausgehenden und vorzugsweise zu diesem verschwenkbaren Flügeln sowie eine nach Gebrauch der Kanüle den distalen Abschnitt abdeckende Schutzeinrichtung, die verschwenkbar an der Handhabe angelenkt und um eine senkrecht zum proximalen Kanülenabschnitt sowie parallel zu einer von den in einer Ebene verlaufenden Flügeln aufgespannten Ebene verlaufende Achse schwenkbar ist und einen kanalförmigen Abschnitt zur Aufnahme des distalen Abschnitts der Kanüle aufweist und bei von dem kanalförmigen Abschnitt aufgenommenen distalen Kanülenabschnitt dieser in dem kanalförmigen Abschnitt fixiert ist wobei die Schutzeinrichtung in Nutzposition der Kanüle in einer sich entlang dem Mittelabschnitt der Handhabe erstreckenden Stellung fixiert ist.

Entsprechende abgewinkelte Kanülen mit flügelartiger Handhabe sind als Portkanülen bekannt, die dazu bestimmt sind, einen in einem Körper implantierten Port mit Medikamenten zu versorgen. Hierzu weist der Port eine Membran mit einer Dicke z. B. zwischen 3 mm und 5 mm und einem Durchmesser von beispielhaft 10 mm bis 20 mm auf, die von dem distalen Abschnitt der Kanüle durchstochen werden muss, um sodann das Medikament zuzuführen. Dabei wird der proximale Abschnitt mittels der Flügel auf der Haut des Patienten fixiert. Der proximale Abschnitt ist mit einem abklemmbaren Schlauch verbunden, der endseitig z. B. einen Luer-Adapter mit Drehverschluss aufweisen kann, um eine Verbindung zu einem Medikamentenbehältnis zu ermöglichen.

Um nach Gebrauch der Kanüle, also nach dem Entfernen aus dem Körper die die Kanüle handhabende Person zu schützen, wird der distale Abschnitt von einer Schutzeinrichtung abgedeckt, die nach der US-B-6,500,155 durch die Flügel der Handhabe selbst gebildet wird. So weisen die Flügel in Bezug auf den distalen, also abgewinkelten Abschnitt der Kanüle, eine Positionierung und eine Erstreckung auf, dass der distale Abschnitt zwischen den Flügeln fixiert wird, die ihrerseits untereinander durch Rastmittel wie Rastvorsprung und Rastausnehmung verbunden werden. Um dimensionsmäßig gleiche Flügel für unterschiedliche Kanülenlängen zu verwenden, die bis zu 40 mm lang sein können, muss jeder Flügel selbst eine Längenerstreckung von in etwa 45 mm aufweisen. Diese Dimensionierung führt zu einer relativ großen Auflagefläche der Flügel auf der Haut, wenn das Medikament dem Port zugeführt wird. Infolgedessen entstehen relativ große Reizzonen, die unangenehm sind.

Der JP-A-2007195827 ist eine Portkanüle zu entnehmen, bei der ein flexibles Rohr über den distalen Abschnitt zu dessen Abdeckung geschoben werden kann. Bei Nutzung der Kanüle wird das Rohr in einen entlang des proximalen Abschnitts verlaufenden rohrförmigen Abschnitt einer Handhabe positioniert.

Eine Portkanüle nach der DE-T-699 35 347 weist gleichfalls eine Flügelanordnung als Handhabe auf, die entlang des proximalen Abschnitts der Kanüle verschiebbar ist, um mit einem äußeren Abschnitt die Spitze des distalen Abschnittes der Kanüle umgeben zu können.

Die US-A-2006/0030825 sieht eine Schutzeinrichtung für einen distalen Abschnitt einer Portkanüle vor, die als gesondertes Teil ausgebildet ist und nach Gebrauch der Kanüle diese rastend umgibt.

Eine Kanülenanordnung der eingangs genannten Art ist der US-A-6,921,388 zu entnehmen, die eine Portkanüle mit einer Schutzkappe beschreibt, die verschwenkbar ist, um einen Schutz nach deren Gebrauch zu ermöglichen. Dabei weist die Schutzkappe einen den distalen Abschnitt der Kanüle aufnehmenden im Schnitt U-förmigen Abschnitt auf, von dessen Innenwandung Rastvorsprünge ausgehen, die bei abgedeckter Kanüle von dieser hintergriffen werden.

Die WO-A-2002/45574 bezieht sich auf eine Portkanüle mit Schutzkappe. Die Schutzkappe ist in Längsrichtung der Kanüle verschiebbar, um die Kanülenspitze bei Nichtbenutzung abzudecken. Endseitig weist die Schutzkappe eine tellerförmige Erweiterung auf, die ein Verstellen der Schutzkappe zum Abdecken der Kanüle erleichtert. Die tellerförmige Erweiterung ist mit der Schutzkappe gelenkig verbunden.

Eine gattungsgemäße Portkanüle ist der EP-A-1 256 355 zu entnehmen. Eine die Kanüle umgebende Schutzeinrichtung ist nach einem Ausführungsbeispiel über eine Feder mit dem Grundkörper einer Handhabe verbunden. In Nutzposition der Kanüle erstreckt sich die Schutzeinrichtung auf der Seite des Grundkörpers, die der Kanüle zugewandt ist.

Eine lösbar mit einem Grundkörper einer Handhabe verbindbare Schutzeinrichtung nach der WO-A-2005/000377 ist ohne Verrastung verschwenkbar zu der Handhabe ausgebildet. Die WO-A-2005/120624 sieht eine Schutzeinrichtung für eine Portkanüle vor, die in einer Hülse verschiebbar angeordnet ist, die ihrerseits zu einem Grundkörper einer Handhabe verschwenkbar ist.

Der vorliegenden Erfindung liegt die Aufgabe zu Grunde, eine Kanülenanordnung der eingangs genannten Art so weiterzubilden, dass bei konstruktiv einfachem Aufbau und sicherer Abdeckung des distalen Abschnitts der Kanüle nach dessen Gebrauch bei der Benutzung der Kanüle eine Behinderung durch die Abdeckung unterbleibt. Auch soll nach einem weiteren Aspekt der Erfindung eine Vereinfachung der Handhabung der Kanüle selbst beim Einstechen des Ports ermöglicht werden, also der distale Abschnitt zielsicher auf den Port ausrichtbar sein.

Zur Lösung der Aufgabe sieht die Erfindung im Wesentlichen vor, dass der kanalförmige Abschnitt der Schutzeinrichtung eine Bodenwandung aufweist, deren Außenseite in Nutzposition der Kanüle auf oberer Außenfläche des Mittelabschnitts der Handhabe aufliegt, dass die Bodenwandung der Schutzeinrichtung auf dem Mittelabschnitt der Handhabe fixiert ist, und dass die Bodenwandung ein erstes Rastmittel aufweist, dem ein in dem Mittelabschnitt der Handhabe vorhandenes zweites Rastmittel zum Verrasten des kanalförmigen Abschnitts zugeordnet ist.

Erfindungsgemäß ist die Schutzeinrichtung derart verschwenkbar und fixierbar, dass eine Behinderung bei der Nutzung der Portkanüle ausgeschlossen ist. Hierzu erstreckt sich die Schutzkappe entlang der Handhabe, so dass die Schutzkappe nicht stört.

Die Bodenwandung wann zumindest in einem Längsbereich parallel zu der Achse verlaufen und das Rastmittel aufweisen.

Auch besteht die Möglichkeit, dass die Schutzeinrichtung mit der Handhabe über ein Scharnier verbunden ist, mittels dessen die Schutzeinrichtung in Nutzposition in einer Totpunktlage und entlang der Handhabe verlaufend fixierbar ist.

Die Erfindung sieht vor, dass der kanalförmige Abschnitt um eine senkrecht zum proximalen Kanülenabschnitt und parallel zu einer von den unverschwenkten Flügeln aufgespannten Ebene verlaufenden Achse schwenkbar ist und dass die Bodenwandung des kanalförmigen Abschnitts zumindest in einem Längsbereich parallel zu der Achse verläuft und ein erstes Rastmittel aufweist, dem ein in dem Mittelabschnitt der Handhabe vorhandenes zweites Rastmittel zum Verrasten des kanalförmigen Abschnitts an den Mittelabschnitt zugeordnet ist. Insbesondere ist das erste Rastmittel eine Aussparung und das zweite Rastmittel ein Vorsprung oder umgekehrt.

Die Schwenkachse selbst kann durch ein Filmscharnier gebildet sein. Dabei verläuft das Filmscharnier zwischen dem Mittelabschnitt der Handhabe und einer proximalen flächigen Erweiterung der Schutzeinrichtung, die in die Bodenwandung des kanalförmigen Abschnitts übergeht. Die flächige Erweiterung weist dabei quer zur Längsachse des proximalen Kanülenabschnitts eine größere Erstreckung als die Bodenwandung auf.

In der Nutzposition der Kanüle verlaufen die Bodenwandung und die flächige Erweiterung entlang dem Mittelabschnitt der Handhabe und sind auf diesem fixiert, wobei die Längsachse des distalen Kanülenabschnitts die flächige Erweiterung durchsetzt. Insbesondere durchsetzt bei entlang dem Mittelabschnitt ausgerichteter Schutzeinrichtung die Längsachse die flächige Erweiterung mittig oder in etwa mittig.

Insbesondere stellt die flächige Erweiterung sicher, dass der abgewinkelte Abschnitt der Kanüle, also der distale Abschnitt präzise auf den Port ausgerichtet werden kann, um sodann in diesen eingestochen zu werden.

Die erfindungsgemäße Schutzeinrichtung ist mit der Handhabe gelenkig verbunden. Somit können die Flügel der Handhabe optimal dimensioniert sein, um Reizungen an der Haut bei gesetzter Kanüle zu vermeiden, da eine geometrische Anpassung an die Längenerstreckung des abzudeckenden distalen Kanülenabschnitts nicht erforderlich ist. Gleichzeitig ist sichergestellt, dass die benutzte Kanüle sicher von der Schutzeinrichtung aufgenommen wird, die dann, wenn sich der distale Kanülenabschnitt in dieser befindet, nicht unkontrolliert verschwenkt werden kann, wodurch anderenfalls die Kanüle wieder freigelegt wäre.

Bevorzugterweise zeigt der kanalförmige Abschnitt senkrecht zur Längsachse betrachtet die Geometrie eines U, wobei der kanalförmige Abschnitt endseitig geschlossen sein sollte. Andere Geometrien sind jedoch gleichfalls möglich. So kann der kanalförmige Abschnitt eine offene kreisähnliche Geometrie, insbesondere eine solche eines Kreisabschnittes aufweisen. Unabhängig von der Geometrie ist vorgesehen, dass der innere Vorsprung in Richtung der Bodenfläche beweglicher als in Richtung der Öffnungsseite des kanalförmigen Abschnitts ist, wodurch das Einbringen in den Raum zwischen dem Vorsprung und der Bodenfläche erleichtert und eine Schwenkbewegung verhindert wird.

Der zumindest eine innere Vorsprung geht von einer ersten Seiteninnenfläche des kanalförmigen Abschnitts aus und ist zumindest abschnittsöffhungsseitig in Richtung der Bodenfläche des kanalförmigen Abschnitts geneigt. Hierdurch ist ein problemloses Einbringen des Kanülenabschnitts in die Schutzeinrichtung möglich, da der Kanülenabschnitt über den Vorsprung in den Bereich zwischen diesem und der Bodenwandung des kanalförmigen Abschnitts geführt wird. Des Weiteren sollte der innere Vorsprung entlang der Bodenfläche und quer zur Längsachse des kanalförmigen Abschnitts betrachtet zur gegenüberliegenden zweiten Seitenfläche und/oder zu einem von dieser ausgehenden zweiten inneren Vorsprung einen lichten Abstand aufweisen, der kleiner als Durchmesser des distalen Kanülenabschnitts ist. Durch diese Maßnahmen ist sichergestellt, dass ein ungewolltes Entfernen des distalen Kanülenabschnittes aus der Schutzeinrichtung dann nicht mehr möglich ist, wenn ersterer in dem Bereich zwischen Vorsprung und Bodenwandung verläuft.

Insbesondere sieht die Erfindung vor, dass zwei Paare von inneren Vorsprüngen vorgesehen sind, wobei einer der Vorsprünge eines jeden Paares von der ersten Seiteninnenfläche und der andere Vorsprung von der zweiten Seiteninnenfläche ausgehen.

Die Vorsprünge weisen in etwa eine zahnartige Struktur mit außenseitig zur Bodenfläche hin geneigter Oberfläche auf.

Des Weiteren sollte der innere Vorsprung in Richtung der Bodenfläche beweglicher als in Richtung Öffnungsseite des kanalförmigen Abschnitts sein, so dass beim Abdecken des distalen Kanülenabschnitts beim Verschwenken der Kanüle in Richtung der Bodenfläche ein geringer Widerstand durch die Vorsprünge entsteht, jedoch ein großer dann herrscht, wenn auf die Schutzeinrichtung, d. h. den kanalförmigen Abschnitt bei in diesem fixierten Kanülenabschnitt eine Kraft einwirkt, die ein Zurückschwenken ermöglichen könnte.

Weitere Einzelheiten, Vorteile und Merkmale der Erfindung ergeben sich nicht nur aus den Ansprüchen, den diesen zu entnehmenden Merkmalen - für sich und/oder in Kombination -, sondern auch aus der nachfolgenden Beschreibung von einem der Zeichnung zu entnehmenden bevorzugten Ausführungsbeispielen.

Es zeigen:
- Fig. 1: eine erfindungsgemäße Kanülenanordnung im Herstellungszustand in Drauf- sicht,
- Fig. 2: die Kanülenanordnung gemäß Fig. 1 in Unteransicht,
- Fig. 3: die Kanülenanordnung gemäß Fig. 1 und 2 in Seitenansicht,
- Fig. 4: die Kanülenanordnung gemäß Fig. 1 bis 3 in perspektivischer Darstellung von oben betrachtet,
- Fig. 5: die Kanülenanordnung gemäß Fig. 1 bis 4 in perspektivischer Darstellung von unten betrachtet,
- Fig. 6: eine Seitenansicht der Kanülenanordnung der Fig. 1 bis 5 im Nutzzustand,
- Fig. 7: eine perspektivische Darstellung der Kanülenanordnung gemäß Fig. 6 in per- spektivischer Darstellung von oben betrachtet,
- Fig. 8: die Kanülenanordnung gemäß der Fig. 6 und 7 mit Schlauch, Schlauchklem- me und Luer-Adapter,
- Fig. 9: die Kanülenanordnung der Fig. 1 bis 8 nach Gebrauch,
- Fig. 10: die Kanülenanordnung nach Gebrauch in Seitenansicht,
- Fig. 11: die Kanülenanordnung nach Gebrauch in perspektivischer Darstellung be- trachtet von unten und
- Fig. 12: die Kanülenanordnung nach Gebrauch mit Schlauch, Schlauchklemme und Luer-Adapter.

In den Figuren, in denen gleiche Elemente mit gleichen Bezugszeichen versehen sind, ist eine Kanülenanordnung 10 dargestellt, die eine Portkanüle 12 mit Handhabe 14 sowie Schutzeinrichtung 16 umfasst. Die Portkanüle 12 umfasst einen proximalen Abschnitt 18 und einen rechtwinklig zu diesem verlaufenden distalen Abschnitt 20, der distal eine geschliffene Spitze 22 aufweist, die in einen in einem Körper implantierten Port einstechbar ist, um ein Medikament zuzuführen.

An das Ende des proximalen Abschnitts 18 der Kanüle 12 ist in gewohnter Weise ein Schlauch 24 aufschiebbar, der seinerseits durch eine Schlauchklemme 26 absperrbar bzw. freigebbar ist. Am Ende des Schlauchs 24 befindet sich im Ausfühmngsbeispiel ein Luer-Adapter 28 mit Drehverschluss.

Die Handhabe 14 weist einen den proximalen Abschnitt 18 der Kanüle 12 umgebenden mittleren Abschnitt 30 auf, der im Ausführungsbeispiel quaderförmig ausgebildet ist. Von den Seitenflächen 32, 34 des Mittelabschnitts 30 gehen Flügel 36, 38 aus, die vorzugsweise zu dem Mittelabschnitt 30 verschenkbar sind, ohne dass dies ein zwingendes Merkmal darstellt. Unterseitig sind die Flügel 36, 38 strukturiert, um beim Fixieren der Kanüle 12 auf einer Patientenhaut eine flächige Auflage zu vermeiden.

Im Ausführungsbeispiel ist integral mit der Handhabe 14 die Schutzeinrichtung 16 ausgebildet wie gespritzt, wobei zwischen dem Mittelabschnitt 30 und der Schutzeinrichtung 16 zum Verschwenken dieser ein Filmscharnier 40 verläuft, das sich senkrecht zu der Kanüle 12, also sowohl zu dem proximalen Abschnitt 38 als auch zu dem distalen Abschnitt 20 erstreckt. Ferner verläuft das Filmscharnier 40 parallel zu einer Ebene, die durch die Flügel 36, 38 in unverschwenktem Zustand aufgespannt ist, wie dies den Figuren zu entnehmen ist. Das Filmscharnier 40 gibt die Schwenkachse vor, die daher auch mit dem Bezugszeichen 40 gekennzeichnet wird, um die ein kanalförmiger Abschnitt 42 der Schutzeinrichtung 16 zu der Handhabe 14 hin bzw. weg von dieser verschwenkbar ist, wie nachstehend erläutert wird.

Der kanalförmige Abschnitt 42 ist im Ausführungsbeispiel im Schnitt U-förmig und endseitig geschlossen (Stirnwandung 44) ausgebildet. Folglich weist der kanalförmige Abschnitt 42 Seitenwandungen 46, 48 sowie eine Bodenwandung 50 auf. Der kanalförmige Abschnitt 42, d. h. dessen Bodenwandung 50 geht in eine flächige Erweiterung 52 über, die über das Filmscharnier 40 mit dem mittleren Abschnitt 30 der Handhabe 14 verbunden ist.

Die flächige Erweiterung 52 ist auf ihrer Unterseite 54 strukturiert, um beim Setzen der Kanüle 12, d. h. Einstechen des distalen Abschnitts 20 der Kanüle als Positionierhilfe zu dienen. So steht dem Nutzer beim Setzen, bei dem die Flügel 36, 38 aufeinander liegen, um ein Hantieren zu ermöglichen, eine relativ große Fläche zur Verfügung, um den distalen Abschnitt 20 führen zu können, damit ein sicheres Einstechen der Spitze 22 in den Port ermöglicht wird.

Von den Innenflächen der Seitenwandung 46, 48 und des kanalförmigen Abschnitts 42 ragen paarweise angeordnete Vorsprünge 56, 58 bzw. 60, 62 ab, die beabstandet zur Innenfläche 64 der Bodenwandung 50 verlaufen und zu diesem einen lichten Abstand aufweisen, der es ermöglicht, dass in dem so gebildeten Zwischenraum der distale Abschnitt 20 der Kanüle 12 positionierbar und über die Vorsprünge 56, 58 bzw. 60, 62 fixierbar ist.

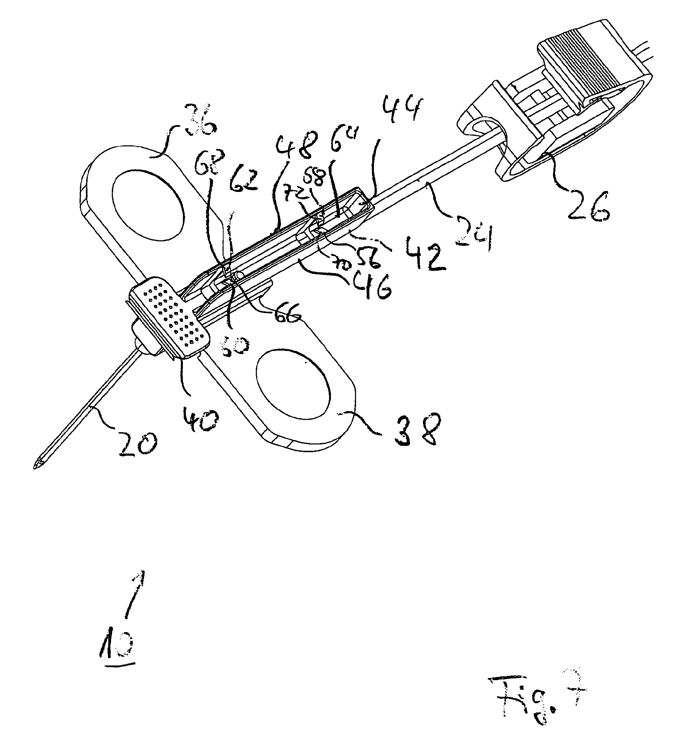

Wie insbesondere aus der Fig. 7 ersichtlich ist, sind die äußeren, also öffnungsseitig verlaufenden Oberseiten 66, 68, 70, 72 der Vorsprünge 56, 58, 60, 62 in Richtung der Bodenfläche 64 geneigt, um ein Einführen des distalen Abschnitts 20 zu erleichtern. Bodenflächenseitig sollten die Vorsprünge 56, 58, 60, 62 in etwa parallel zur Bodenfläche verlaufen, so dass die Vorsprünge, 56, 58, 60, 62 wandseitig eine größere Stärke als endseitig aufweisen. Unabhängig hiervon sollten die Vorsprünge 56, 58, 60, 62 so dimensioniert und angeordnet sein, dass beim Einwirken einer Kraft in Richtung der Bodenfläche 64 eine größere Beweglichkeit gegeben ist als bei entgegengerichtet wirkender Kraft.

Des Weiteren verläuft in der Bodenwandung 50 des kanalförmigen Abschnitts 42 eine Aussparung 74, der ein von dem mittleren Abschnitt 30 der Handhabe 14 senkrecht abragende Vorsprung 76 zugeordnet ist.

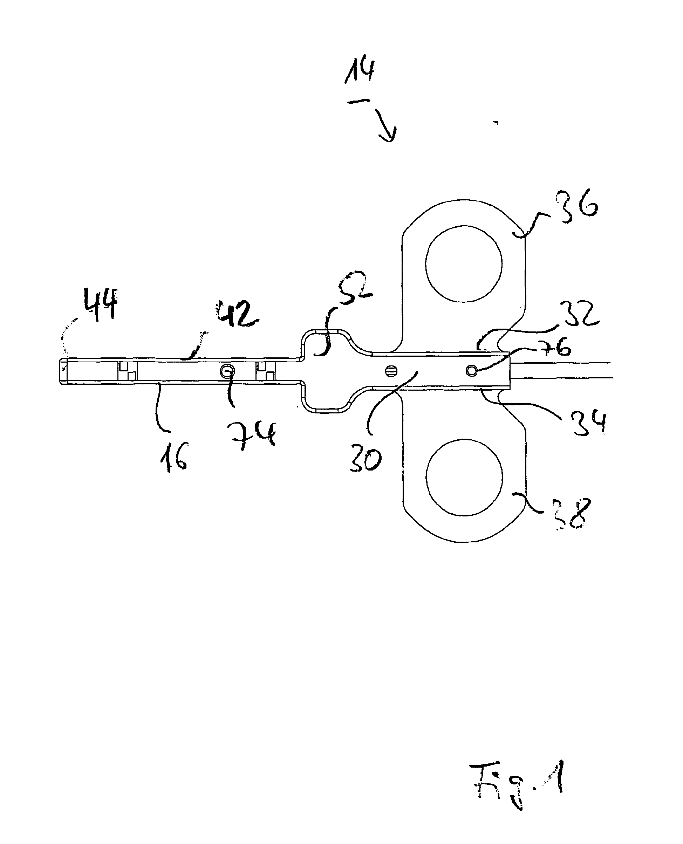

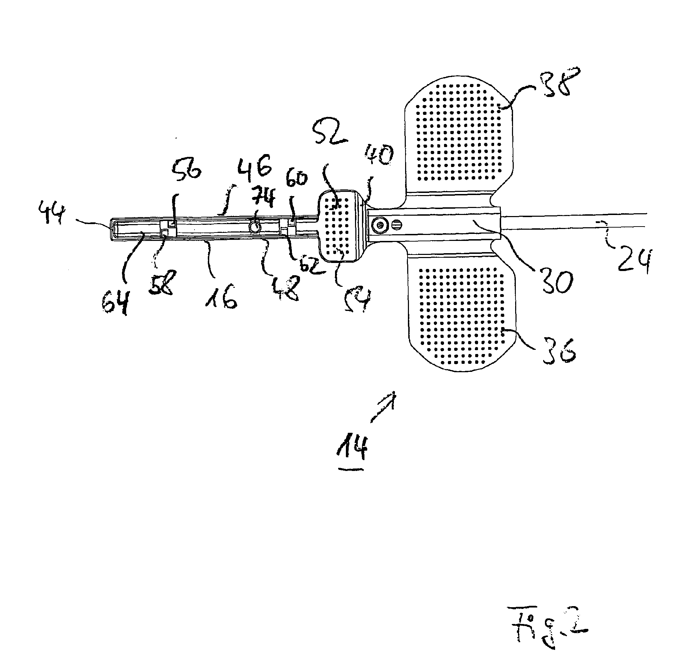

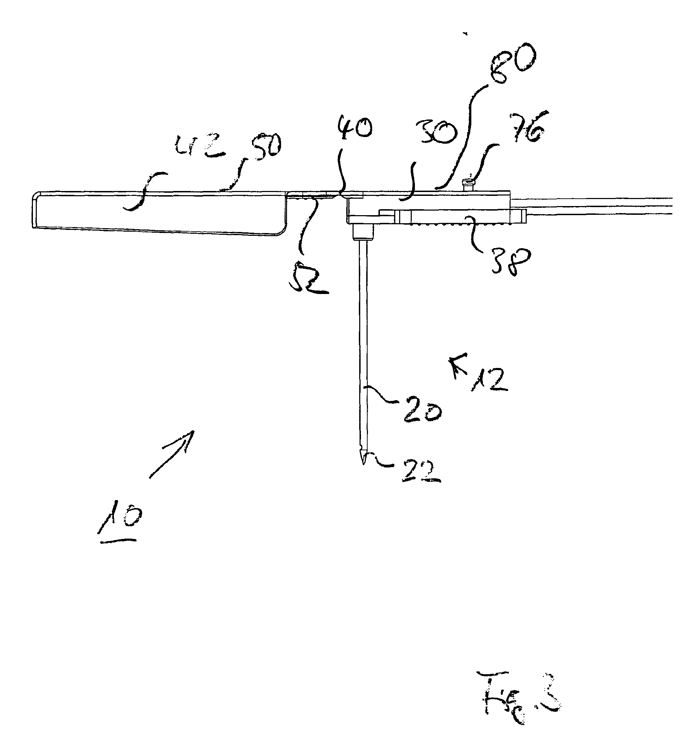

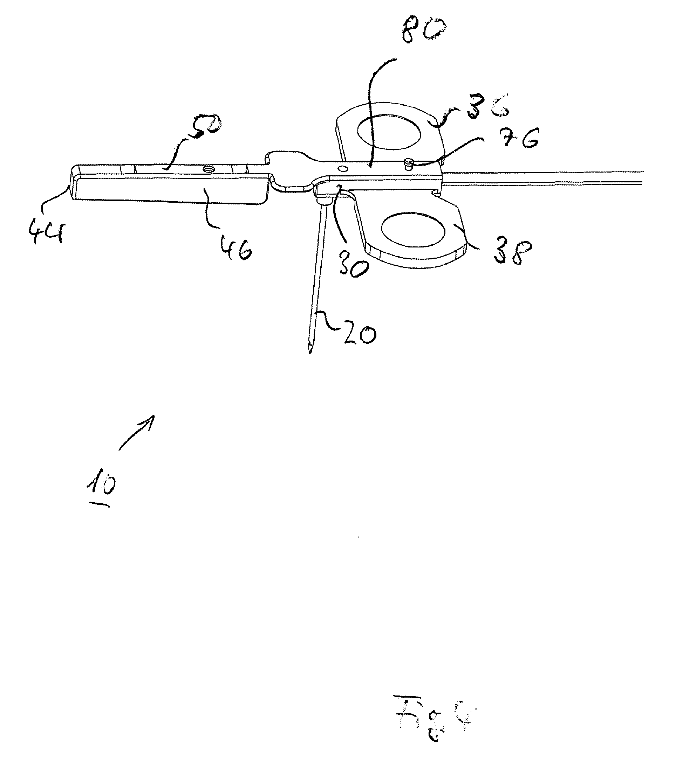

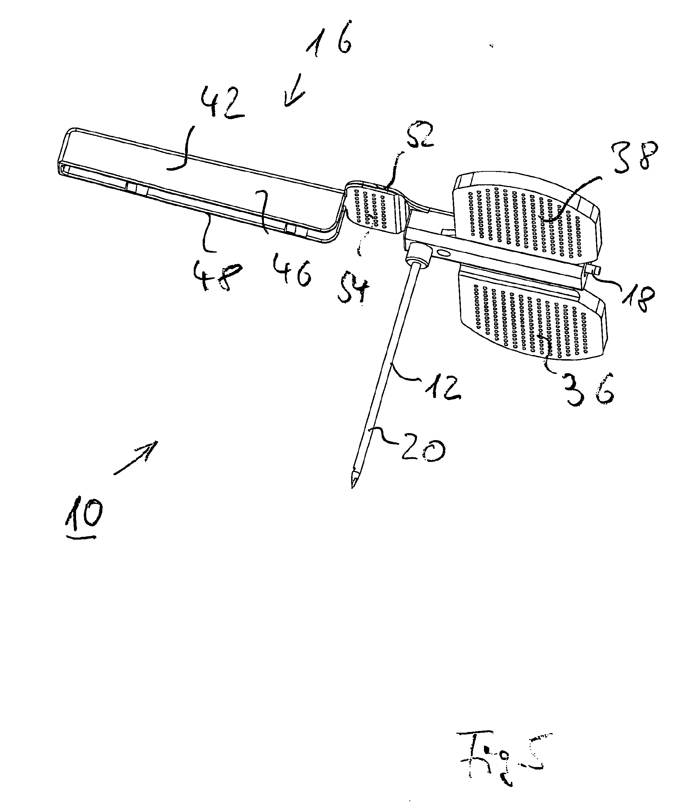

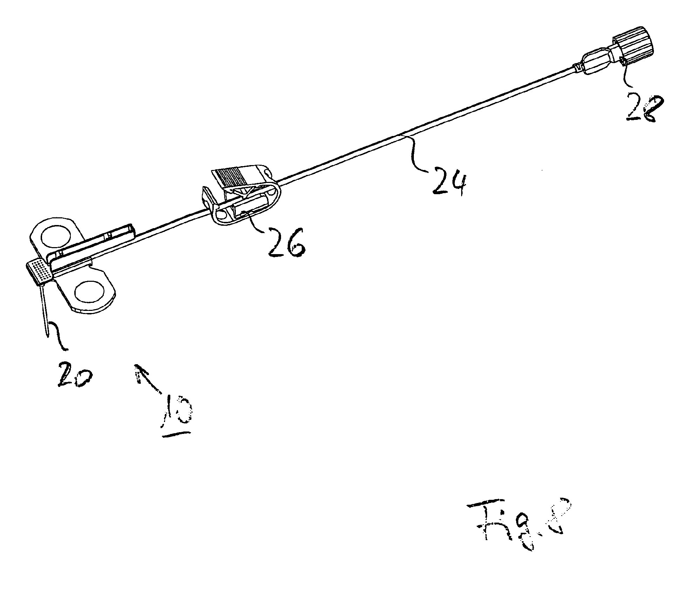

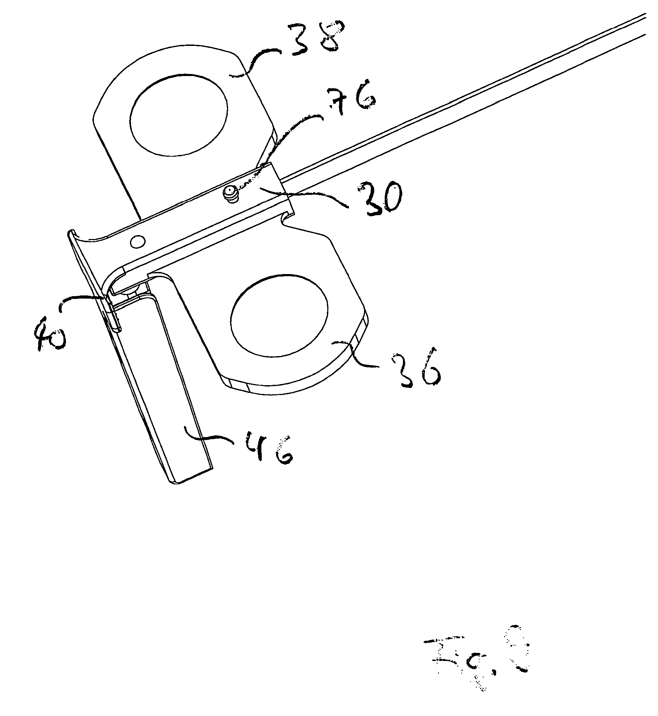

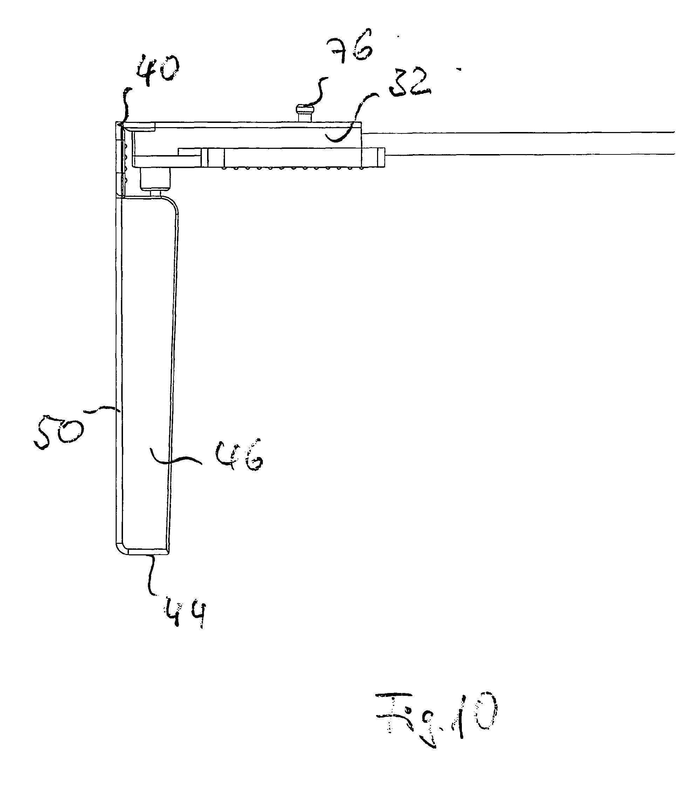

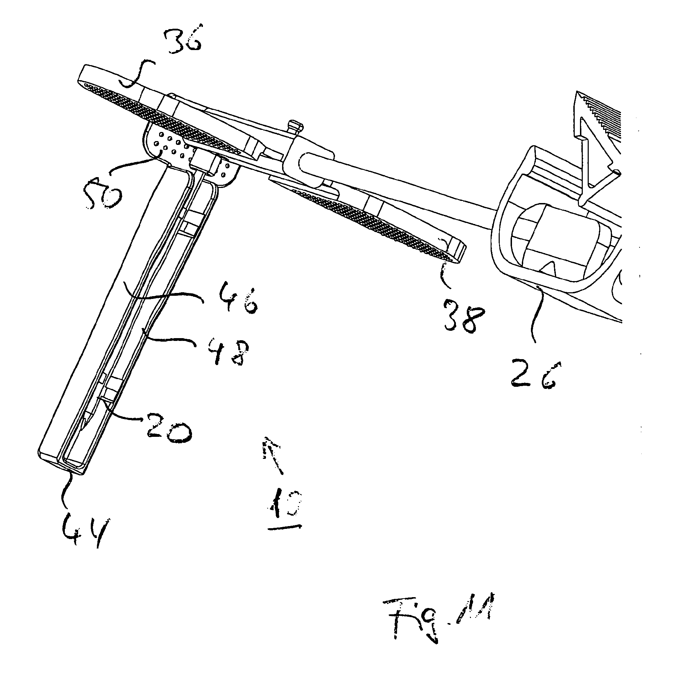

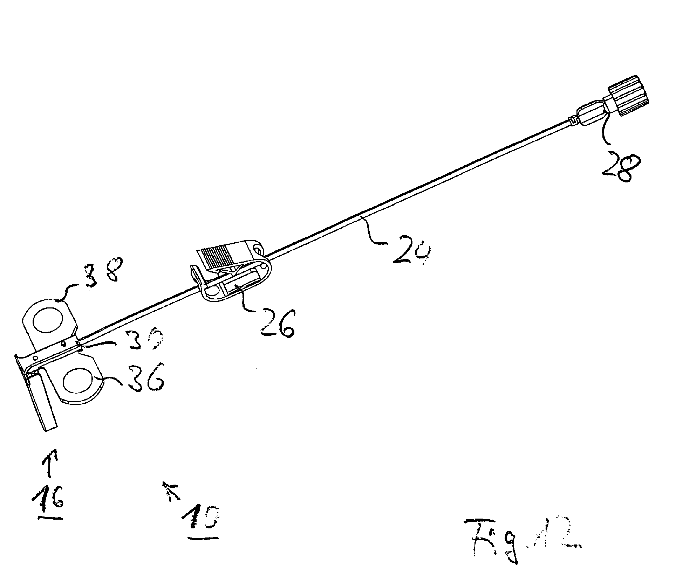

Die Funktion der Schutzeinrichtung 16 bzw. deren Positionierung bei der Herstellung, im Nutzzustand sowie nach der Benutzung der Kanüle 12 ergeben sich aus den Fig. 1 bis 5 bzw. 6 bis 8 bzw. 9 bis 12.

So ist in den Fig. 1 bis 5 die Schutzeinrichtung 16 im Herstellungszustand dargestellt, bei dem die Schutzeinrichtung 16 zusammen mit der Handhabe 14 die Kanüle 12, d. h. deren proximaler Abschnitt 18 und bereichsweise der distale Abschnitt 20 umspritzt werden. In diesem Zustand verläuft die Längsachse der Schutzreinrichtung 16 entlang der von den Flügeln 36, 38 gemeinsam aufgespannten Ebene.

Soll die Kanülenanordnung 10 genutzt werden, so wird die Schutzeinrichtung 16 um das die Schwenkachse vorgebende Filmscharnier 40 in Richtung der Handhabe 14 verschwenkt, damit der Vorsprung 76, der von der Außenseite des mittleren oder Mittelabschnitts 30 der Handhabe 14 abragt, einrasten kann, so dass eine Behinderung durch die Schutzeinrichtung 16 bei der Nutzung der Kanülenanordnung 10 nicht erfolgen kann.

Anstelle einer entsprechenden Rastverbindung kann die Schutzeinrichtung 16 mit der Handhabe 14 auch über ein Scharnier mit sogenannter Totpunktlage verbunden werden. Dies bedeutet, dass dann, wenn die Schutzeinrichtung 16 zu den Flügeln 36, 38 verschwenkt ist, das Scharnier eine Totpunktlage einnimmt und somit die Schutzeinrichtung 16 nicht unkontrolliert verschwenkt werden kann.

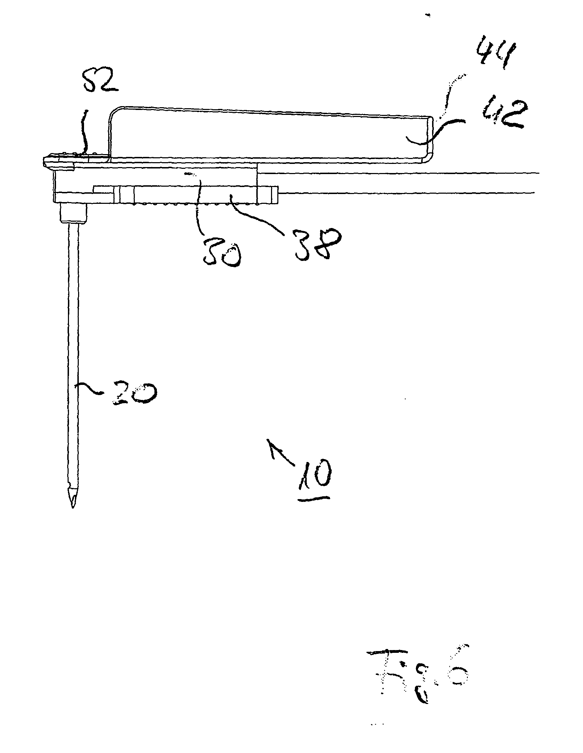

Der Nutzzustand der Kanülenanordnung 10 ergibt sich aus den Fig. 6 bis 8. Man kann erkennen, dass der als Schutzkappe zu bezeichnende kanalförmige Abschnitt 42 der Schutzeinrichtung 16 mit der Außenseite 78 der Bodenwandung 50 auf der oberen Außenfläche 80 des Mittelabschnittes 30 der Handhabe 14 liegt, wobei der Vorsprung 76 in die Aussparung 74 eingreift. Auch die flächige Erstreckung 52 der Schutzeinrichtung 16 erstreckt sich entlang der Fläche 80, wie die Fig. 6 und 7 verdeutlichen. Dabei ist die strukturierte Fläche 54 der Erstreckung 52 außenliegend. Ferner sind das Filmscharnier 40 und die flächige Erstreckung 52 derart angeordnet bzw. ausgebildet, dass letztere von dem distalen Abschnitt 20 der Kanüle 12 durchsetzt wird. Gegebenenfalls kann ein mittiges Durchsetzen erfolgen.

Soll die Portkanüle nunmehr gesetzt werden, werden die Flügel 36, 38 zusammengeklappt, um also bereichsweise aufeinanderzuliegen, um die Kanülenanordnung 10 auf einen Bereich eines Patienten auszurichten, in dem ein Einstechen in einen Port erfolgen kann. Die flächige Erstreckung 52 dient dabei als Manövrierhilfe, so dass positionsgenaues Ausrichten des distalen Abschnitts 22 auf den Port, d. h. zum Durchstechen der Membran erfolgen kann. Ist die Kanüle gesetzt, so werden die Flügel 36, 38 zurückalso auseinandergeklappt und auf der Haut des Patienten mittels eines Pflasters fixiert. Da die Dimensionierung, d. h. flächige Erstreckung der Flügel 36, 38 ausschließlich auf die sichere Handhabung der Kanülenanordnung 10 und nicht auf die Länge des distalen Abschnitts 20 abgestimmt sein muss, kann eine relativ geringe Flächenerstreckung gewählt werden, wodurch eine Reizung der Haut vermindert wird.

Nachdem das Medikament über den distalen Abschnitt 20 zugeführt worden ist, wird die Kanüle 12 entfernt und sodann die Schutzeinrichtung 16 um das Filmscharnier 40 in den zeichnerischen Darstellungen entgegen dem Uhrzeigersinn verschwenkt, bis der distale Abschnitt 20 die Öffnung des kanalförmigen Abschnitts 42 durchsetzt und die Vorsprünge 56, 58, 60, 62 überwindet, um in den Zwischenraum zwischen diesen und der Bodenfläche 64 des kanalförmigen Abschnitts 42 zu gelangen. In dieser Position ist der distale Abschnitt 20 fixiert, da auch dann, wenn auf den kanalförmigen Abschnitt 42 eine Kraft zum Verschwenken um das Filmscharnier 40, also in den zeichnerischen Darstellungen im Uhrzeigersinn erfolgen sollte, die Vorsprünge 56, 58, 60, 62 aufgrund der Dimensionierung und Gestaltung nicht überwunden werden können, so dass eine sichere Abdeckung des benutzten distalen Kanülenabschnitts 20 gewährleistet ist.

Es erfolgt ein Verrasten zwischen der Bodenfläche 64 und den Vorsprüngen 56, 58, 60, 62, deren Erstreckung quer zur Längsachse des Abschnitts 42 derart ist, dass der vorhandene Freiraum zwischen den Vorsprüngen 56, 58, 60, 62 bzw. eines Vorsprungs und der Seitenwandung, von der der Vorsprung nicht ausgeht, so abgestimmt ist, dass ein lichter Abstand gegeben ist, der kleiner als Durchmesser des distalen Abschnitts der Kanüle 12 ist.

Anstelle eines im Schnitt U-förmigen kanalförmigen Abschnitts 42 kann auch eine andere Geometrie gewählt werden. Gleiches gilt bezüglich der Vorsprünge und deren Gestaltung, ohne dass die Erfindung verlassen wird. Ausschlaggebend ist allein, dass nach Benutzen der Kanüle 12 deren distaler Abschnitt 20 sicher in der Schutzeinrichtung 16, d. h. dem entsprechend gestalteten kanalförmigen Abschnitt 42 fixiert ist, ohne dass ein ungewolltes Verschwenken dieses erfolgen kann, da der verrastete distale Abschnitt 20 ein solches verhindert. Anstelle eines Verrastens kann auch ein Festklemmen erfolgen.

## Patentansprüche

1. Kanülenanordnung (10) umfassend eine Kanüle (12) mit einem proximalen Abschnitt (18) und einem distalen Abschnitt (20), die zueinander einen rechten oder einen im Wesentlichen rechten Winkel einschließen, eine von dem proximalen Abschnitt ausgehende Handhabe (14) umfassend einen den proximalen Abschnitt aufnehmenden Mittelabschnitt (30) mit von diesem ausgehenden und vorzugsweise zu diesem verschwenkbaren Flügeln (36, 38) sowie eine nach Gebrauch der Kanüle den distalen Abschnitt abdeckende Schutzeinrichtung (16),
die verschwenkbar an der Handhabe (14) angelenkt und um eine senkrecht zum proximalen Kanülenabschnitt (18) sowie parallel zu einer von den in einer Ebene verlaufenden Flügeln (36, 38) aufgespannten Ebene verlaufende Achse (40) schwenkbar ist und einen kanalförmigen Abschnitt (42) zur Aufnahme des distalen Abschnitts (20) der Kanüle (12) aufweist und bei von dem kanalförmigen Abschnitt aufgenommenen distalen Kanülenabschnitt dieser in dem kanalförmigen Abschnitt fixiert ist, wobei
die Schutzeinrichtung in Nutzposition der Kanüle (12) in einer sich entlang dem Mittelabschnitt (30) der Handhabe (14) erstreckenden Stellung fixiert ist,
**dadurch gekennzeichnet,**
**dass** der kanalförmige Abschnitt (42) der Schutzeinrichtung (16) eine Bodenwandung (50) aufweist, deren Außenseite (78) in Nutzposition der Kanüle (12) auf oberer Außenfläche (80) des Mittelabschnitts (30) der Handhabe (14) aufliegt, dass
die Bodenwandung (50) der Schutzeinrichtung (16) auf dem Mittelabschnitt (30) der Handhabe (14) fixiert ist, und dass die Bodenwandung (50)
ein erstes Rastmittel (74) aufweist, dem ein in dem Mittelabschnitt (30) der Handhabe (14) vorhandenes zweites Rastmittel (76) zum Verrasten des kanalförmigen Abschnitts zugeordnet ist.

2. Kanülenanordnung nach Anspruch 1,
**dadurch gekennzeichnet,**
**dass** die Bodenwandung (50) zumindest in einem Längsbereich parallel zu der Achse verläuft und das Rastmittel (74) aufweist.

3. Kanülenanordnung nach Anspruch 1 oder 2,
**dadurch gekennzeichnet,**
**dass** das erste Rastmittel eine Aussparung (74) und das zweite Rastmittel ein Vorsprung (76) ist oder umgekehrt.

4. Kanülenanordnung nach zumindest einem der vorhergehenden Ansprüche,
**dadurch gekennzeichnet,**
**dass** die Achse (40) durch ein Filmscharnier gebildet ist,
das zwischen dem Mittelabschnitt (30) der Handhabe (14) und einer proximalen flächigen Erweiterung (52) der Schutzeinrichtung (16) verläuft, die in die Bodenwandung (50) der Schutzeinrichtung übergeht und quer zum proximalen Kanülenabschnitt (18) vorzugsweise eine größere Erstreckung als die Bodenwandung (50) aufweist.

5. Kanülenanordnung nach zumindest einem der vorhergehenden Ansprüche,
**dadurch gekennzeichnet,**
**dass** bei auf dem Mittelabschnitt (30) der Handhabe (14) fixiertem bzw. entlang diesem sich erstreckendem kanalförmigen Abschnitt (42) dessen Bodenwandung (50) und die flächige Erstreckung (52) entlang Oberseite des Mittelabschnitts (30) bzw. auf dieser liegend verläuft.

6. Kanülenanordnung nach zumindest einem der vorhergehenden Ansprüche,
**dadurch gekennzeichnet,**
**dass** bei entlang der Außenfläche des Mittelabschnitts (30) verlaufender flächiger Erweiterung (52) diese von der Längsachse des distalen Kanülenabschnitts (20) durchsetzt ist.

7. Kanülenanordnung nach zumindest einem der vorhergehenden Ansprüche, **dadurch gekennzeichnet,**
**dass** die Längsachse des distalen Kanülenabschnitts (20) die flächige Erstreckung (52) mittig oder in etwa mittig durchsetzt.

8. Kanülenanordnung nach zumindest einem der vorhergehenden Ansprüche,
**dadurch gekennzeichnet,**
**dass** der kanalförmige Abschnitt (42) zumindest einen inneren Vorsprung (56, 58, 60, 62) aufweist, der bei in dem kanalförmigen Abschnitt fixiertem distalen Kanülenabschnitt (20) von diesem hintergriffen ist, wobei der zumindest eine innere Vorsprung (56, 58, 60, 62) von einer ersten Innenseitenfläche des kanalförmigen Abschnitts (42) ausgeht und zumindest abschnittsöffnungsseitig in Richtung Bodenfläche (64) des kanalförmigen Abschnitts (42) geneigt verläuft.

9. Kanülenanordnung nach zumindest einem der vorhergehenden Ansprüche,
**dadurch gekennzeichnet,**
**dass** der zumindest eine innere Vorsprung (56, 58, 60, 62) entlang der Bodenfläche und quer zur Längsachse des kanalförmigen Abschnitts (42) betrachtet zur gegenüberliegenden zweiten Innenfläche und/oder zu einem von dieser ausgehenden zweiten inneren Vorsprung einen lichten Abstand aufweist, der kleiner als Durchmesser des distalen Kanülenabschnitts (20) ist.

10. Kanülenanordnung nach zumindest einem der vorhergehenden Ansprüche,
**dadurch gekennzeichnet,**
**dass** zwei Paar von inneren Vorsprüngen (56, 58, 60, 62) vorgesehen sind, wobei einer der Vorsprünge eines Paares von der ersten Seiteninnenfläche und der andere Vorsprung von der zweiten Seiteninnenfläche ausgeht.

11. Kanülenanordnung nach zumindest einem der vorhergehenden Ansprüche,
**dadurch gekennzeichnet,**
**dass** der innere Vorsprung (56, 58) in Richtung der Bodenfläche (64) beweglicher als in Richtung der Öffnungsseite des kanalförmigen Abschnitts (42) ist.

12. Kanülenanordnung nach zumindest einem der vorhergehenden Ansprüche,
**dadurch gekennzeichnet,**
**dass** der kanalförmige Abschnitt (42) senkrecht zur Längsachse betrachtet im Schnitt eine U-Form aufweist oder eine offene kreisähnliche Geometrie aufweist.

## Claims

1. Cannula arrangement (10) comprising a cannula (12) with a proximal section (18) and a distal section (20), which enclose a right angle or a substantially right angle to each other, a handle (14) emanating from the proximal section and comprising a central section (30) accommodating the proximal section with wings (36, 38) emanating from said central section (30) and preferably pivotable thereto, and a protective device (16) covering the distal section after use of the cannula, said protective device (16) being pivotably articulated on the handle (14) and pivotable about an axis (40) that is perpendicular to the proximal cannula section (18) and parallel to a plane formed by the wings (36, 38) extending in one plane and having a channel-shaped section (42) to accommodate the distal section (20) of the cannula (12) and with the distal cannula section fixed in the channel-shaped section when it is accommodated by said channel-shaped section and with the protective device fixed in a position extending along the central section (30) of the handle (14) in the position of use of the cannula (12),
wherein
the channel-shaped section (42) of the protective device (16) has a base wall (50) whose outer side (78) in the position of use of the cannula (12) contacts the upper outer surface (80) of the central section (30) of the handle (14), the base wall (50) of the protective device (16) is fixed on the central section (30) of the handle (14), and the base wall (50) has a first catch means (74) to which is assigned a second catch means (76) provided in the central section of the handle (14) for locking of the channel-shaped section.

2. Cannula arrangement according to Claim 1,
wherein
the base wall (50) runs parallel to the axis at least in a longitudinal region and has the catch means (74).

3. Cannula arrangement according to Claim I or 2,
wherein
the first catch means is a recess (74) and the second catch means is a projection (76), or vice versa.

4. Cannula arrangement according to at least one of the preceding claims,
wherein
the axis (40) is formed by a film hinge that runs between the central section (30) of the handle (14) and a proximal flat extension (52) of the protective device (16) which then merges into the base wall (50) of the protective device and preferably has a greater extension than the base wall (50) transversely to the proximal cannula section (18).

5. Cannula arrangement according to at least one of the preceding claims,
wherein
when the channel-shaped section (42) is fixed on the central section (30) of the handle (14) or extends along it its base wall (50) and the flat extension (52) runs along the upper side of the central section (30) or contacting it.

6. Cannula arrangement according to at least one of the preceding claims,
wherein
when the flat extension (52) runs along the outer surface of the central section (30) it is intersected by the longitudinal axis of the distal cannula section (20).

7. Cannula arrangement according to at least one of the preceding claims,
wherein
the longitudinal axis of the distal cannula section (20) intersects the flat extension (52) centrally or approximately centrally.

8. Cannula arrangement according to at least one of the preceding claims,
wherein
the channel-shaped section (42) has at least one internal projection (56, 58, 60, 62) which is engaged from behind by the distal cannula section (20) when the latter is fixed in the channel-shaped section, where the at least one inner projection (56, 58, 60, 62) emanates from a first inner side surface of the channel-shaped section (42) and extends inclined at least on the section opening side in the direction of the base surface (64) of the channel-shaped section (42).

9. Cannula arrangement according to at least one of the preceding claims,
wherein
the at least one inner projection (56, 58, 60, 62), viewed along the base surface and transversely to the longitudinal axis of the channel-shaped section (42), has a clear distance from the opposite second inner surface and/or a second inner projection emanating therefrom, said distance being smaller than the diameter of the distal cannula section (20).

10. Cannula arrangement according to at least one of the preceding claims,
wherein
two pairs of inner projections (56, 58, 60, 62) are provided, where one of the projections of one pair emanates from the first side inner surface and the other projection from the second side inner surface.

11. Cannula arrangement according to at least one of the preceding claims,
wherein
the inner projection (56, 58) is more movable in the direction of the base surface (64) than in the direction of the opening side of the channel-shaped section (42).

12. Cannula arrangement according to at least one of the preceding claims,
wherein
the channel-shaped section (42), viewed perpendicularly to the longitudinal axis, has in section a U-shape or an open circle-like geometry.

## Revendications

1. Système de canule (10) comprenant une canule (12) avec une partie proximale (18) et une partie distale (20) qui inscrivent entre elles un angle droit ou quasiment droit, une manette (14) partant de la partie proximale comprenant une partie médiane (30) logeant la partie proximale avec des ailettes (36, 38) partant de celle-ci et de préférence pivotantes par rapport à celle-ci, ainsi qu'un dispositif protecteur (16) recouvrant la partie distale après usage de la canule, lequel est fixé de manière articulée à la manette (14), peut pivoter autour d'un axe (40) perpendiculaire à la partie proximale (18) de la canule et parallèle à un plan défini par les ailettes (36, 38) s'étendant dans un plan, et présente une partie (42) en forme de conduit destinée à loger la partie distale (20) de la canule (12), et lorsque la partie distale de la canule se loge dans la partie en forme de conduit, ladite partie distale est fixée dans la partie en forme de conduit, sachant qu'en position d'utilisation de la canule (12), le dispositif protecteur est fixé dans une position s'étendant le long de la partie médiane (30) de la manette (14),
**caractérisé en ce**
**que** la partie en forme de conduit (42) du dispositif protecteur (16) présente une paroi de fond (50) dont la face extérieure (78) en position d'utilisation de la canule (12) s'appuie sur la face extérieure supérieure (80) de la partie médiane (30) de la manette (14), que la paroi de fond (50) du dispositif protecteur (16) est fixée sur la partie médiane (30) de la manette (14), et que la paroi de fond (50) présente un premier moyen de crantage (74) auquel est associé un second moyen de crantage (76) présent dans la partie médiane (30) de la manette (14) afin d'enclencher la partie en forme de conduit.

2. Système de canule selon la revendication 1,
**caractérisé en ce**
**que** la paroi de fond (50) s'étend parallèlement à l'axe au moins dans une zone longitudinale et présente le moyen de crantage (74).

3. Système de canule selon la revendication 1 ou 2,
**caractérisé en ce**
**que** le premier moyen de crantage est un évidement (74) et le second moyen de crantage est une partie saillante (76), ou inversement.

4. Système de canule selon au moins une des revendications précédentes,
**caractérisé en ce**
**que** l'axe (40) est formé par un film-charnière qui s'étend entre la partie médiane (30) de la manette (14) et un élargissement proximal en nappe (52) du dispositif protecteur (16), qui se prolonge dans la paroi de fond (50) du dispositif protecteur et présente transversalement à la partie proximale (18) de la canule de préférence une plus grande extension que la paroi de fond (50).

5. Système de canule selon au moins une des revendications précédentes,
**caractérisé en ce**
**que** lorsque la partie en forme de conduit (42) est fixée sur la partie médiane (30) de la manette (14) ou s'étend le long de celle-ci, sa paroi de fond (50) et l'extension en nappe (52) s'étendent le long de la face supérieure de la partie médiane (30) ou s'appuient sur celle-ci.

6. Système de canule selon au moins une des revendications précédentes,
**caractérisé en ce**
**que** lorsque l'élargissement en nappe (52) s'étend le long de la face extérieure de la partie médiane (30), il est traversé par l'axe longitudinal de la partie distale de la canule (20).

7. Système de canule selon au moins une des revendications précédentes,
**caractérisé en ce**
**que** l'axe longitudinal de la partie distale de la canule (20) traverse l'extension en nappe (52) en son milieu ou approximativement en son milieu.

8. Système de canule selon au moins une des revendications précédentes,
**caractérisé en ce**
**que** la partie en forme de conduit (42) présente au moins une partie saillante intérieure (56, 58, 60, 62) qui, lorsque la partie distale de la canule (20) est fixée dans la partie en forme de conduit, est en prise arrière avec celle-ci, sachant que l'au moins une partie saillante intérieure (56, 58, 60, 62) part d'une première face latérale intérieure de la partie en forme de conduit (42) et s'étend de manière inclinée au moins du côté de l'ouverture en direction de la face inférieure (64) de la partie en forme de conduit (42).

9. Système de canule selon au moins une des revendications précédentes,
**caractérisé en ce**
**que**, considérée le long de la face inférieure et transversalement à l'axe longitudinal de la partie en forme de conduit (42), l'au moins une partie saillante intérieure (56, 58, 60, 62) présente, par rapport à la deuxième face intérieure opposée et/ou à une deuxième partie saillante intérieure partant de celle-ci, un écartement qui est inférieur au diamètre de la partie distale (20) de la canule.

10. Système de canule selon au moins une des revendications précédentes,
**caractérisé en ce**
**que** sont prévues deux paires de parties saillantes intérieures (56, 58, 60, 62), sachant que l'une des parties saillantes d'une paire part de la première face latérale intérieure et l'autre partie saillante de la seconde face latérale intérieure.

11. Système de canule selon au moins une des revendications précédentes,
**caractérisé en ce**
**que** la partie saillante intérieure (56, 58) est plus mobile en direction de la face inférieure (64) qu'en direction du côté ouvert de la partie en forme de conduit (42).

12. Système de canule selon au moins une des revendications précédentes,
**caractérisé en ce**
**que**, considérée perpendiculairement à l'axe longitudinal, la partie en forme de conduit (42) présente une section transversale en forme de U ou à géométrie quasi circulaire ouverte.
